# EUROPEAN PATENT SPECIFICATION

(11) **EP 4 359 410 B1**
(45) Date of publication and mention of the grant of the patent: **12.02.2025**
(21) Application number: 22736251.4
(22) Date of filing: 23.06.2022
(51) Int. Cl.: C07D 471/04, A61P 35/00, A61K 31/519

(54) **CRYSTALLINE FORM OF SOTORASIB**
KRISTALLINE FORM VON SOTORASIB
FORME CRISTALLINE DE SOTORASIB

(30) Priority: 24.06.2021 EP 21181306; 10.02.2022 EP 22156069
(43) Date of publication of application: 01.05.2024
(73) Proprietor: SANDOZ AG, 4051 Basel (CH)
(72) Inventor: ADAMER, Verena, 6250 Kundl (AT); MARGREITER, Renate, 6250 Kundl (AT); PICHLER, Arthur, 6250 Kundl (AT)
(74) Representative: Prüfer & Partner mbB Patentanwälte · Rechtsanwälte
(86) International application number: PCT/EP2022/067276
(87) International publication number: WO 2022/269008

(56) References cited:
- WO-A1-2020/236947

## Description

### FIELD OF THE INVENTION

The present invention relates to a crystalline form of sotorasib and to a process for its preparation. Furthermore, the invention relates to a pharmaceutical composition comprising the crystalline form of sotorasib of the present invention and at least one pharmaceutically acceptable excipient. The pharmaceutical composition of the present invention can be used as a medicament, in particular for the treatment of a cancer with a KRAS mutation.

### BACKGROUND OF THE INVENTION

Sotorasib is a selective inhibitor of the KRAS G12C mutant useful in the treatment of cancers, including treatment of lung cancer, such as non-small cell lung cancer (NSCLC), pancreatic cancer and colorectal cancer. The chemical name of sotorasib is 6-fluoro-7-(2-fluoro-6-hydroxyphenyl)-1-(4-methyl-2-(2-propanyl)-3-pyridinyl)-4-((2*S*)-2-methyl-4-(2-propenoyl)-1-piperazinyl)pyrido[2,3-d]pyrimidin-2(1*H*)-one. Sotorasib can be represented by the following chemical structure according to Formula (A)

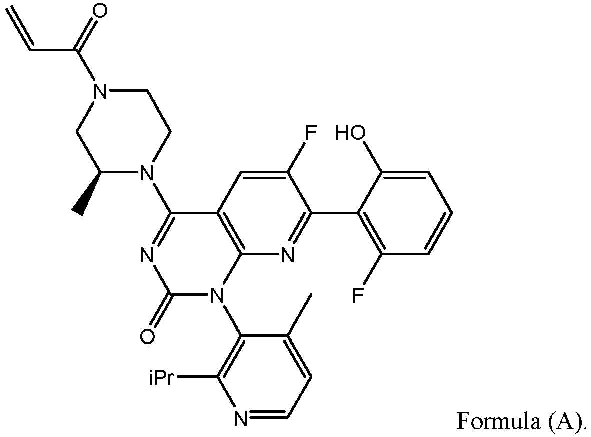

Sotorasib and its preparation are disclosed in WO 2018/217651 A1 *e.g.* in Example 41. The last step includes purification by silica gel chromatography. The application is silent about the obtained solid-state form though.

WO 2020/236947 A1 describes various crystalline forms of sotorasib including several anhydrous forms designated Form I, Form II and Form III, a hydrate form and several solvates, which were the result of an extensive polymorph screening program.

WO 2021/236920 A1 discloses various crystalline forms of sotorasib including anhydrous forms as well as several hydrates and solvates.

Different solid-state forms of an active pharmaceutical ingredient often possess different properties. Differences in physicochemical properties of solid-state forms can play a crucial role for the improvement of pharmaceutical compositions, for example, pharmaceutical formulations with improved dissolution profile and bioavailability or with improved stability or shelf-life can become accessible due to an improved solid-state form of an active pharmaceutical ingredient. Also processing or handling of the active pharmaceutical ingredient during the formulation process may be improved. New solid-state forms of an active pharmaceutical ingredient can thus have desirable processing properties. They can be easier to handle, better suited for storage, and/or allow for better purification, compared to previously known solid forms.

There is thus a need for the provision of crystalline forms of sotorasib having improved physicochemical properties.

In particular, there is a need to provide an improved crystalline form of sotorasib, which is physically stable *e.g.* does not undergo phase transformations during standard pharmaceutical processing and storage, which is non-hygroscopic or only slightly hygroscopic and at the same time shows high solubility and dissolution rates in physiologically relevant aqueous media.

### SUMMARY OF THE INVENTION

The present invention provides a crystalline form of sotorasib, which is hereinafter also designated as "Form A".

Sotorasib Form A of the present invention posesses one or more advantageous properties selected from the group consisting of chemical stability, physical stability, melting point, hygroscopicity, solubility, dissolution, morphology, crystallinity, flowability, bulk density, compactibility and wettability.

For example, the crystalline Form A of the present invention is physically stable against temperature and humidity stress which is important for the reliable manufacture and stable storage of a pharmaceutical composition comprising sotorasib Form A (see Examples 7, 8, 9 and 10 as well as Comparative Example 1 hereinafter).

In addition, Form A of the present invention is more soluble in physiologically relevant aqueous media such as acetate buffer pH 4.5 and phosphate buffer pH 6.8 compared to crystalline form I of sotorasib disclosed in WO 2020/236947 A1 (see Comparative Example 2 hereinafter) which is a crucial advantage for a low soluble drug substance like sotorasib in order to reach the desired *in vivo* blood levels.

This unique combination of advantageous properties renders the crystalline Form A of the present invention the preferred solid form of sotorasib for the preparation of a pharmaceutical composition comprising sotorasib.

### Abbreviations

- PXRD: powder X-ray diffractogram
- FTIR: Fourier transform infrared
- DSC: differential scanning calorimetry
- TGA: thermogravimetric analysis
- GMS: gravimetric moisture sorption
- MTBE: methyl tert-butyl ether
- DIPE: diisopropyl ether
- w-%: weight percent
- RH: relative humidity
- RT: room temperature
- rpm: rotations per minute

### Definitions

In the context of the present invention the following definitions have the indicated meaning, unless explicitly stated otherwise:

The term "sotorasib" as used herein, means 6-fluoro-7-(2-fluoro-6-hydroxyphenyl)-1-(4-methyl-2-(2-propanyl)-3-pyridinyl)-4-((2*S*)-2-methyl-4-(2-propenoyl)-1-piperazinyl)pyrido[2,3-*d*]pyrimidin-2(1*H*)-one. Sotorasib can be represented by the chemical structure according to Formula (A).

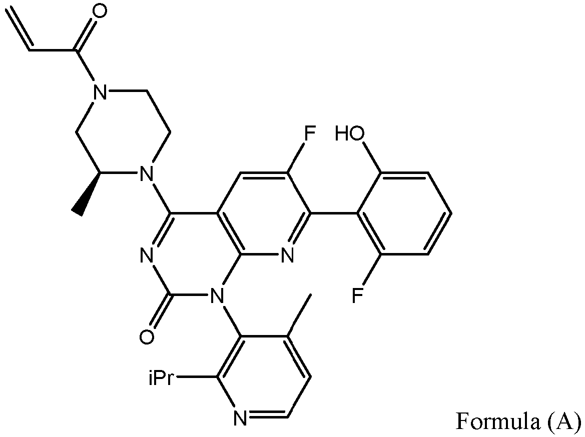

Sotorasib may exist as atropisomers, which are conformational stereoisomers that occur when rotation about a single bond in the molecule is prevented, or greatly slowed, as a result of steric interactions with other parts of the molecule.

For example, sotorasib may be present as atropisomer ***M*** as represented by the chemical structure according to Formula (A1)

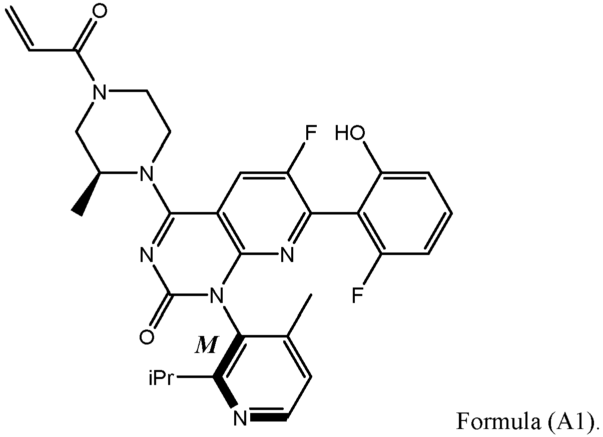

Sotorasib may also be present as atropisomer ***P*** as represented by the chemical structure according to Formula (A2)

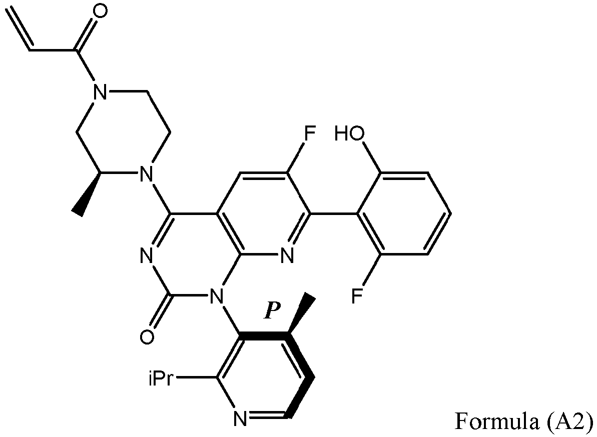

Sotorasib as disclosed herein includes all atropisomers, both as pure individual atropisomers, enriched atropisomers or non-specific mixtures of atropisomers. Most preferably, sotorasib as disclosed herein is present as ***M*** atropisomer.

As used herein, the term "measured at a temperature in the range of from 20 to 30°C" refers to a measurement under standard conditions. Typically, standard conditions mean a temperature in the range of from 20 to 30°C, *i.e.* at room temperature. Standard conditions can mean a temperature of about 22°C. Typically, standard conditions can additionally mean a measurement under 20-60% RH, preferably 30-50% RH, more preferably 40% RH.

As used herein the term "room temperature" refers to a temperature in the range of from 20 to 30°C.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order *e.g*. extends over approximately 100 to 1000 atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 6.4° 2-Theta for example can appear between 6.2° and 6.6° 2-Theta, preferably between 6.3° and 6.5° 2-Theta on most X-ray diffractometers under standard conditions. Furthermore, one skilled in the art will appreciate that relative reflection intensities will show inter-apparatus variability as well as variability due to degree of crystallinity, preferred orientation, particle size, sample preparation and other factors known to those skilled in the art and should be taken as qualitative measure only.

Crystalline Form A of sotorasib of the present invention may be referred to herein as being characterized by graphical data "as shown in" a figure. Such data include, for example, powder X-ray diffraction. The person skilled in the art understands that factors such as variations in instrument type, response and variations in sample directionality, sample concentration and sample purity may lead to small variations for such data when presented in graphical form, for example variations relating to the exact reflection positions and intensities. However, a comparison of the graphical data in the figures herein with the graphical data generated for another or an unknown solid form and the confirmation that two sets of graphical data relate to the same crystal form is well with in the knowledge of a person skilled in the art.

The term "solid-state form" as used herein refers to any crystalline and/or amorphous phase of a compound.

As used herein, the term "amorphous" refers to a solid-state form of a compound that is not crystalline. An amorphous compound possesses no long-range order and does not display a definitive X-ray diffraction pattern with reflections.

The terms "anhydrous" or "anhydrate" as used herein refer to a crystalline solid where no water is cooperated in or accommodated by the crystal structure.

A "predetermined amount" as used herein with regard to sotorasib Form A refers to the initial amount of sotorasib Form A used for the preparation of a pharmaceutical composition having a desired dosage strength of sotorasib.

The term "effective amount" as used herein with regard to sotorasib Form A encompasses an amount of sotorasib Form A which causes the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, disintegrating agent, dissolution modifying agent, absorption enhancer, stabilizer or a manufacturing aid among others. Excipients may include fillers (diluents), binders, disintegrants, lubricants and glidants.

The terms "filler" or "diluent" as used herein refer to substances that are used to dilute the active pharmaceutical ingredient prior to delivery. Diluents and fillers can also serve as stabilizer or disintegrant.

The terms "disintegrant" or "disintegrating agent" as used herein refers to substances which, upon addition to a solid pharmaceutical composition, facilitate its break-up or disintegration after administration and permits the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process.

### BRIEF DESCRIPTION OF THE FIGURES

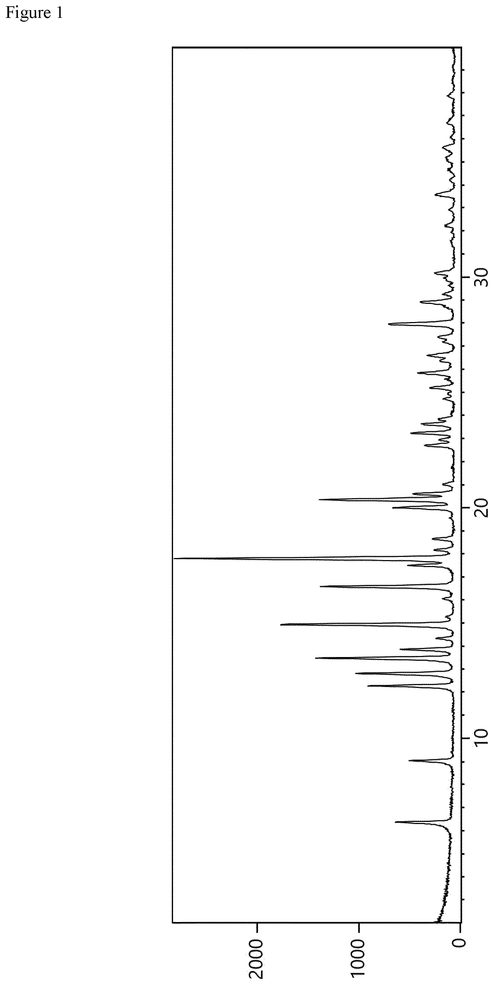

**Figure 1****:** illustrates a representative PXRD of sotorasib Form A according to the present invention. The x-axis shows the scattering angle in °2-Theta, the y-axis shows the intensity of the scattered X-ray beam in counts of detected photons.

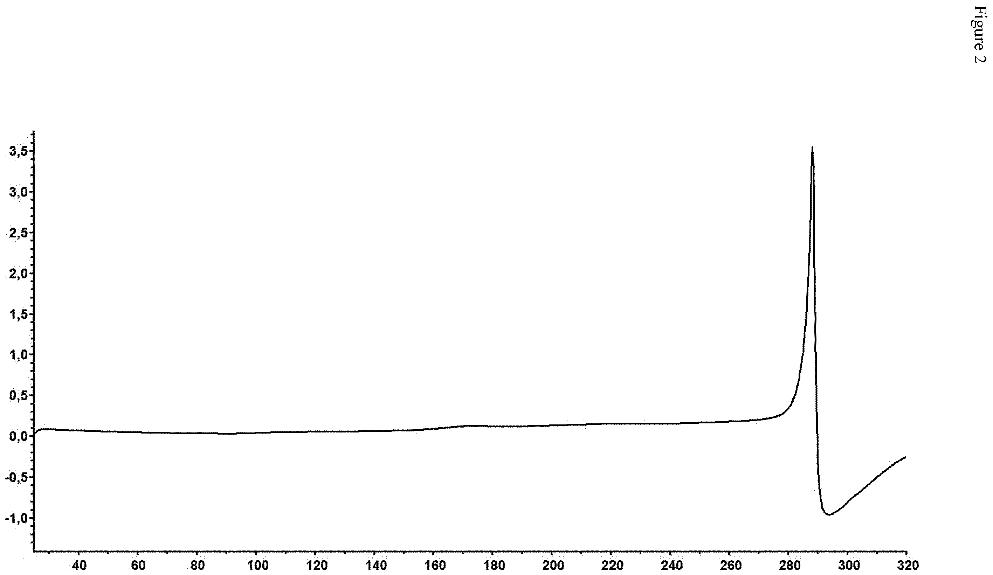

**Figure 2****:** illustrates a DSC curve of sotorasib Form A of the present invention. The x-axis shows the temperature in degree Celsius (°C), the y-axis shows the heat flow rate in Watt per gram (W/g) with endothermic peaks going up.

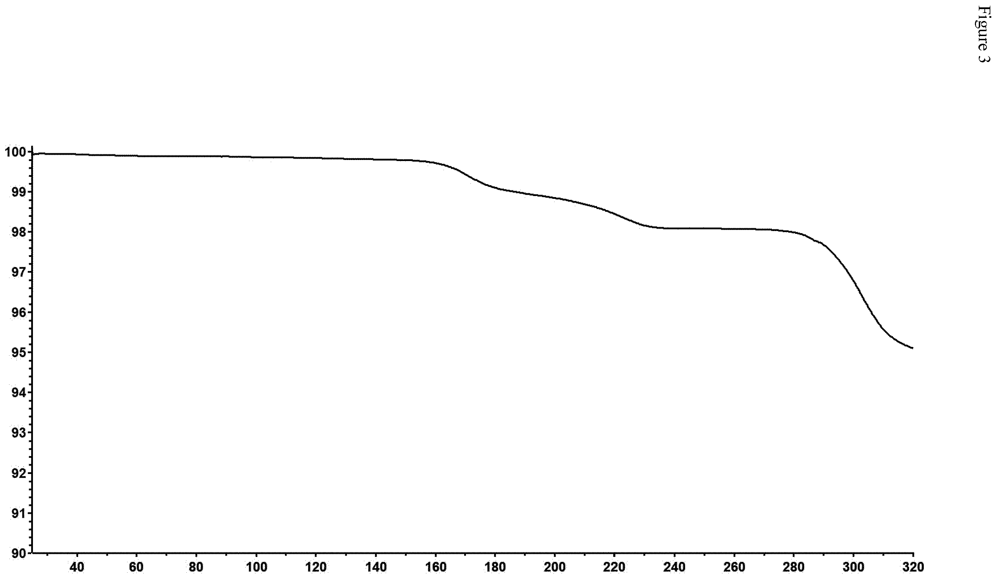

**Figure 3****:** illustrates a TGA curve of sotorasib Form A of the present invention. The x-axis shows the temperature in degree Celsius (°C), the y-axis shows the mass (loss) of the sample in weight percent (w-%).

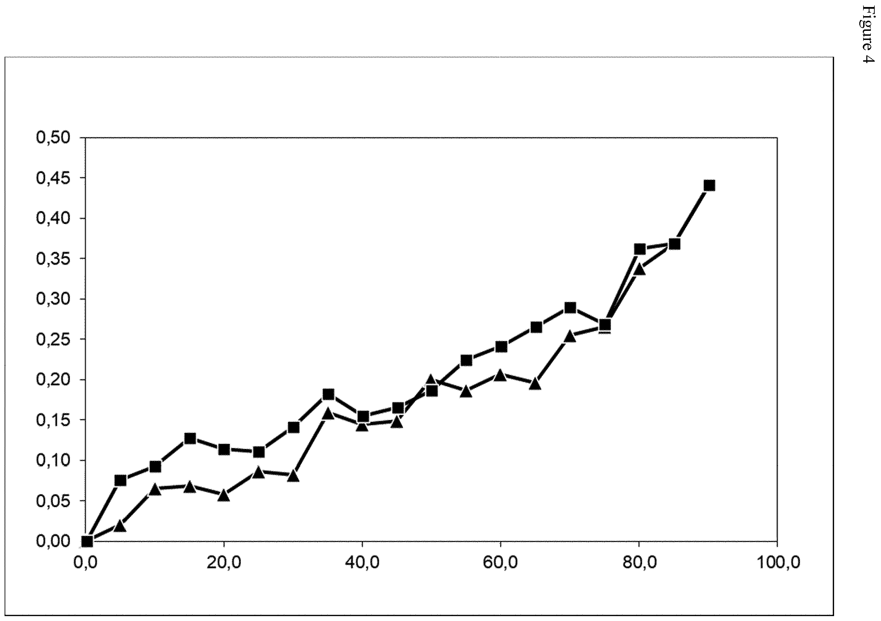

**Figure 4****:** illustrates GMS isotherms of sotorasib Form A of the present invention in the range of from 0 to 90% RH. The x-axis displays the relative humidity in percent (%) measured at a temperature of (25.0 ± 0.1)°C, the y-axis displays the equilibrium mass change in weight percent (w-%). Sample weight at 0% RH at the start of the sorption curve is used as reference weight. Sorption curve points are displayed as triangles, desorption curve points as squares.

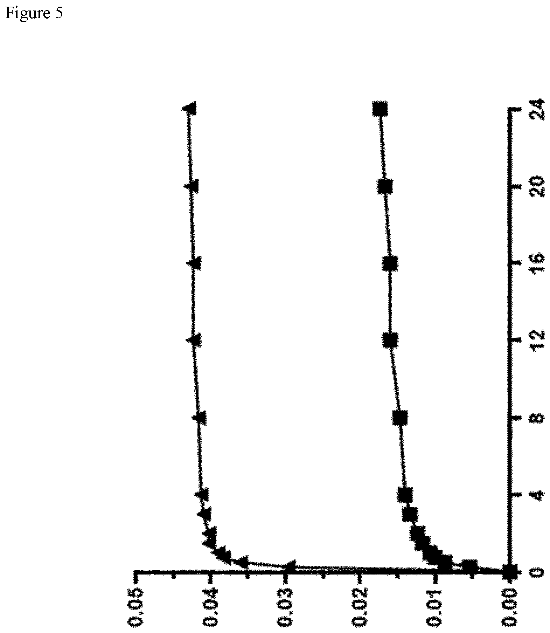

**Figure 5****:** displays the dissolution curves of the crystalline Form I of sotorasib disclosed in WO 2020/236947 A1 (black squares) and of the crystalline Form A of sotorasib of the present invention (black triangles) in acetate buffer pH 4.5 measured at 37°C. The x-axis shows the time in hours, the y-axis the sotorasib concentration of the solution in microgram per millilitre.

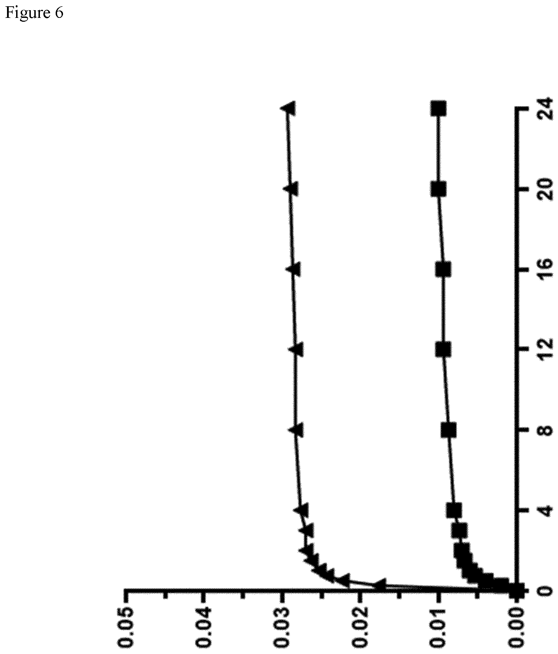

**Figure 6****:** displays the dissolution curves of the crystalline Form I of sotorasib disclosed in WO 2020/236947 A1 (black squares) and of the crystalline Form A of sotorasib of the present invention (black triangles) in phosphate buffer pH 6.8 measured at 37°C. The x-axis shows the time in hours, the y-axis the sotorasib concentration of the solution in microgram per millilitre.

### DETAILED DESCRIPTION OF THE INVENTION

The present invention provides a crystalline form of sotorasib, herein also designated as "Form A".

The crystalline form (Form A) of sotorasib of the present invention may be represented by the chemical structure according to Formula (A)

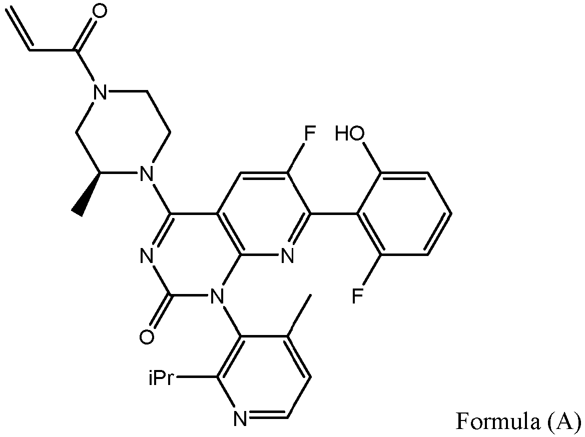

Most preferably, the crystalline form (Form A) of sotorasib is the M atropisomer and may be represented by the chemical structure according to Formula (A1)

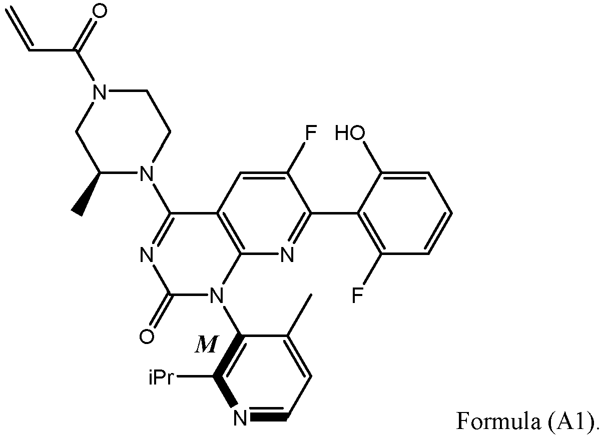

Alternatively, the crystalline form (Form A) of sotorasib may be present as the P atropisomer and may be represented by the chemical structure according to Formula (A2)

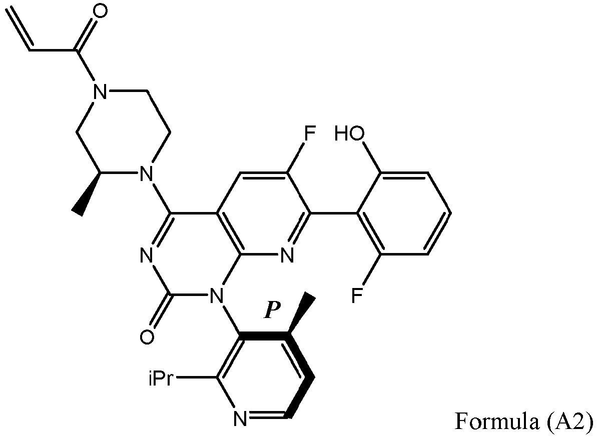

The crystalline Form A of sotorasib of the present invention may be characterized by analytical methods well known in the field of the pharmaceutical industry for characterizing solids. Such methods comprise but are not limited to powder X-ray diffraction, FTIR spectroscopy, DSC, TGA and GMS. Sotorasib Form A of the present invention may be characterized by one of the aforementioned analytical methods or by combining two or more of them. In particular, Form A of sotorasib of the present invention may be characterized by any one of the following embodiments or by combining two or more of the following embodiments.

In one embodiment the invention relates to a crystalline form (Form A) of sotorasib characterized by having a PXRD comprising reflections at 2-Theta angles of:
(6.4 ± 0.2)°, (12.3 ± 0.2)° and (17.8 ± 0.2)°; or
(6.4 ± 0.2)°, (12.3 ± 0.2)°, (16.6 ± 0.2)° and (17.8 ± 0.2)°; or
(6.4 ± 0.2)°, (9.1 ± 0.2)°, (12.3 ± 0.2)°, (16.6 ± 0.2)° and (17.8 ± 0.2)°; or
(6.4 ± 0.2)°, (9.1 ± 0.2)°, (12.3 ± 0.2)°, (15.0 ± 0.2)°, (16.6 ± 0.2)° and (17.8 ± 0.2)°; or
(6.4 ± 0.2)°, (9.1 ± 0.2)°, (12.3 ± 0.2)°, (13.5 ± 0.2)°, (15.0 ± 0.2)°, (16.6 ± 0.2)° and (17.8 ± 0.2)°; or
(6.4 ± 0.2)°, (9.1 ± 0.2)°, (12.3 ± 0.2)°, (12.8 ± 0.2)°, (13.5 ± 0.2)°, (15.0 ± 0.2)°, (16.6 ± 0.2)° and (17.8 ± 0.2)°; or
(6.4 ± 0.2)°, (9.1 ± 0.2)°, (12.3 ± 0.2)°, (12.8 ± 0.2)°, (13.5 ± 0.2)°, (15.0 ± 0.2)°, (16.6 ± 0.2)°, (17.8 ± 0.2)° and (20.4 ± 0.2)°; or
(6.4 ± 0.2)°, (9.1 ± 0.2)°, (12.3 ± 0.2)°, (12.8 ± 0.2)°, (13.5 ± 0.2)°, (13.9 ± 0.2)°, (15.0 ± 0.2)°, (16.6 ± 0.2)°, (17.8 ± 0.2)° and (20.4 ± 0.2)°;
when measured at a temperature in the range of from 20 to 30°C with Cu-Kalpha_{1,2} radiation having a wavelength of 0.15419 nm.

In another embodiment, the invention relates to a crystalline form (Form A) of sotorasib characterized by having a PXRD comprising reflections at 2-Theta angles of (6.4 ± 0.2)°, (12.3 ± 0.2)°, (12.8 ± 0.2)°, (13.5 ± 0.2)°, (15.0 ± 0.2)°, (16.6 ± 0.2)°, (17.8 ± 0.2)°, (20.0 ± 0.2)°, (20.4 ± 0.2)° and (27.9 ± 0.2)°, when measured at a temperature in the range of from 20 to 30°C with Cu-Kalpha_{1,2} radiation having a wavelength of 0.15419 nm.

In a further embodiment, the present invention relates to a crystalline form (Form A) of sotorasib characterized by having a PXRD comprising reflections at 2-Theta angles of:
(6.4 ± 0.1)°, (12.3 ± 0.1)° and (17.8 ± 0.1)°; or
(6.4 ± 0.1)°, (12.3 ± 0.1)°, (16.6 ± 0.1)° and (17.8 ± 0.1)°; or
(6.4 ± 0.1)°, (9.1 ± 0.1)°, (12.3 ± 0.1)°, (16.6 ± 0.1)° and (17.8 ± 0.1)°; or
(6.4 ± 0.1)°, (9.1 ± 0.1)°, (12.3 ± 0.1)°, (15.0 ± 0.1)°, (16.6 ± 0.1)° and (17.8 ± 0.1)°; or
(6.4 ± 0.1)°, (9.1 ± 0.1)°, (12.3 ± 0.1)°, (13.5 ± 0.1)°, (15.0 ± 0.1)°, (16.6 ± 0.1)° and (17.8 ± 0.1)°; or
(6.4 ± 0.1)°, (9.1 ± 0.1)°, (12.3 ± 0.1)°, (12.8 ± 0.1)°, (13.5 ± 0.1)°, (15.0 ± 0.1)°, (16.6 ± 0. 1)° and (17.8 ± 0.1)°; or
(6.4 ± 0.1)°, (9.1 ± 0.1)°, (12.3 ± 0.1)°, (12.8 ± 0.1)°, (13.5 ± 0.1)°, (15.0 ± 0.1)°, (16.6 ± 0.1)°, (17.8 ± 0.1)° and (20.4 ± 0.1)°; or
(6.4 ± 0.1)°, (9.1 ± 0.1)°, (12.3 ± 0.1)°, (12.8 ± 0.1)°, (13.5 ± 0.1)°, (13.9 ± 0.1)°, (15.0 ± 0.1)°, (16.6 ± 0.1)°, (17.8 ± 0.1)° and (20.4 ± 0.1)°;
when measured at a temperature in the range of from 20 to 30°C with Cu-Kalpha_{1,2} radiation having a wavelength of 0.15419 nm.

In still another embodiment, the invention relates to a crystalline form (Form A) of sotorasib characterized by having a PXRD comprising reflections at 2-Theta angles of (6.4 ± 0.1)°, (12.3 ± 0.1)°, (12.8 ± 0.1)°, (13.5 ± 0.1)°, (15.0 ± 0.1)°, (16.6 ± 0.1)°, (17.8 ± 0.1)°, (20.0 ± 0.1)°, (20.4 ± 0.1)° and (27.9 ± 0.1)°, when measured at a temperature in the range of from 20 to 30°C with Cu-Kalpha_{1,2} radiation having a wavelength of 0.15419 nm.

In another embodiment, the invention relates to a crystalline form (Form A) of sotorasib characterized by having a PXRD comprising no reflection at 2-Theta angles in the range of from 6.7° to 8.8°, when measured at a temperature in the range of from 20 to 30°C with Cu-Kalpha_{1,2} radiation having a wavelength of 0.15419 nm.

In yet another embodiment, the invention relates to a crystalline form (Form A) of sotorasib characterized by having a PXRD comprising no reflection at 2-Theta angles in the range of from 9.4° to 12.0°, when measured at a temperature in the range of from 20 to 30°C with Cu-Kalpha_{1,2} radiation having a wavelength of 0.15419 nm.

In a further embodiment, the present invention relates to a crystalline form of sotorasib (Form A) characterized by having a PXRD essentially the same as shown in Figure 1 of the present invention, when measured at a temperature in the range of from 20 to 30°C with Cu-Kalpha_{1,2} radiation having a wavelength of 0.15419 nm.

The PXRD of sotorasib Form A of the present invention can be readily distinguished from the PXRDs of the sotorasib forms disclosed in WO 2020/236947 A1 and WO 2021/236920 A1. The PXRD of Form A for example shows a characteristic reflection at (6.4 ± 0.2)° 2-Theta, whereas except for Form III which shows a reflection at 6.3° 2-Theta, none of the other forms of sotorasib disclosed in WO 2020/236947 A1 show reflections in the same range. On the other hand the PXRD of Form A of the present invention exhibits reflections which are not present in the diffractogram of Form III of WO 2020/236947 A1 *e.g.* at 2-Theta angles of (12.3 ± 0.2)°, (14.4 ± 0.2)° and (23.7 ± 0.2)°.

Form A of the present invention also exhibits a characteristic reflection at (16.6 ± 0.2)° 2-Theta, which is for example not present in the diffractogram of Form 4 of WO 2021/236920 A1.

In another embodiment, the present invention relates to a crystalline form (Form A) of sotorasib, characterized by having a TGA curve showing a mass loss of not more than 2.0 w-%, preferably of not more than 1.9 w-%, such as not more than 1.8 w-%, based on the weight of the crystalline form, when heated from 25 to 250°C at a rate of 10 K/min.

In a further embodiment, the present invention relates to a crystalline form (Form A) of sotorasib characterized by showing a mass change of not more than 2.0 w-%, preferably of not more than 1.0 w-%, most preferably of not more than 0.5 w-%, such as not more than 0.4 w-% based on the weight of the crystalline form, when measured with GMS at a relative humidity in the range of from 0 to 90% and a temperature of (25.0 ± 1.0)°C.

In one embodiment, the present invention relates to a crystalline form (Form A) of sotorasib characterized in being anhydrous.

In another aspect, the present invention discloses a composition comprising the crystalline form (Form A) of sotorasib of the present invention as defined in any one of the above described embodiments, said composition being essentially free of any other solid-state form of sotorasib. For example, a composition comprising the crystalline Form A of sotorasib of the present invention comprises at most 20 w-%, preferably at most 10 w-%, more preferably at most 5 w-%, 4 w-%, 3 w-%, 2 w-% or 1 w-% of any other solid-state form than Form A of sotorasib, based on the weight of the composition. Preferably, the any other solid-state form is selected from the group consisting of crystalline Form I, Form II and Form III of sotorasib of WO 2020/236947 A1 or amorphous sotorasib.

In another embodiment, the invention discloses a composition comprising at least 90 w-%, including at least 90, 91, 92, 93, 94, 95, 96, 97, 98 and 99 w-%, and also including equal to about 100 w-% of the crystalline form (Form A) of sotorasib as defined in any one of the above described embodiments, based on the total weight of the composition. The remaining material may comprise other solid-state form(s) of sotorasib, and/or reaction impurities and/or processing impurities arising from the preparation of the composition.

In another aspect, the present invention relates to a first process for the preparation of the crystalline form (Form A) of sotorasib of the present invention or the composition comprising the same as defined in any one of the above described aspects and their corresponding embodiments comprising:
(a) Dissolving or suspending amorphous sotorasib in a solvent comprising isobutanol, diisopropyl ether and/or methyl *tert*-butyl ether, more preferably in a solvent selected from the group consisting of isobutanol, diisopropyl ether and methyl *tert*-butyl ether or a mixture thereof;
(b) crystallizing sotorasib Form A or the composition comprising the same as defined in any one of the above described aspects and their corresponding embodiments from the solution or suspension obtained in (a) at a temperature of 30°C or less;
(c) separating at least a part of the crystals obtained in (b) from the mother liquor;
(d) optionally washing the isolated crystals obtained in (c); and
(e) drying the crystals obtained in (c) or (d);

In still another aspect, the present invention relates to a second process for the preparation of the crystalline form (Form A) of sotorasib of the present invention or the composition comprising the same as defined in any one of the above described aspects and their corresponding embodiments comprising:
(a) dissolving sotorasib in a solvent comprising methanol, preferably dissolving sotorasib in methanol;
(b) adding a solvent comprising isobutanol, preferably adding isobutanol to the solution obtained in (a);
(c) removing the methanol from the solution obtained in (b);
(d) optionally, seeding the mixture obtained in (c) with sotorasib Form A crystals of the present invention;
(e) crystallizing sotorasib Form A or the composition comprising the same as defined in any one of the above described aspects and their corresponding embodiments from the mixture obtained in (d) at a temperature of 30°C or less;
(f) separating at least a part of the crystals obtained in (e) from the mother liquor;
(g) optionally washing the isolated crystals obtained in (f); and
(h) drying the crystals obtained in (f) or (g);

In a further aspect, the present invention relates to the use of the crystalline form of sotorasib (Form A) of the present invention, or the composition comprising the crystalline form of sotorasib (Form A) of the present invention as defined in any one of the above described aspects and their corresponding embodiments for the preparation of a pharmaceutical composition comprising sotorasib.

In another aspect, the present invention relates to a pharmaceutical composition comprising the crystalline form of sotorasib (Form A) of the present invention, or the composition comprising the crystalline form of sotorasib (Form A) of the present invention as defined in any one of the above described aspects and their corresponding embodiments, preferably in a predetermined and/or effective amount, and at least one pharmaceutically acceptable excipient.

Preferably, the predetermined and/ or effective amount of the crystalline form of sotorasib (Form A) of the present invention, or the composition comprising the crystalline form of sotorasib (Form A) of the present invention as defined in any one of the above described aspects and their corresponding embodiments is in the range of from about 120 to 960 mg, calculated as anhydrous sotorasib. For example, the predetermined and/ or effective amount is selected from the group consisting of 120 mg, 180 mg, 240 mg, 300 mg, 360 mg, 420 mg, 480 mg, 540 mg, 600 mg, 660 mg, 720 mg, 780 mg, 840 mg, 900 mg and 960 mg calculated as anhydrous sotorasib. Preferably, the predetermined and/ or effective amount is selected from the group consisting of 120 mg, 360 mg, 720 mg and 960 mg, calculated as anhydrous sotorasib. Most preferably the predetermined and/ or effective amount is 120 mg, calculated as anhydrous sotorasib.

The at least one pharmaceutically acceptable excipient, which is comprised in the pharmaceutical composition of the present invention, is preferably selected from the group consisting of fillers, disintegrants, binders, lubricants, glidants and combinations thereof. Preferably, the at least one pharmaceutically acceptable excipient, which is comprised in the pharmaceutical composition of the present invention is selected from the group consisting of fillers, disintegrants, lubricants and combinations thereof.

In a particular embodiment, the present invention relates to a pharmaceutical composition as defined in any one of the above described embodiments comprising the crystalline form of sotorasib (Form A) or the composition comprising the crystalline form of sotorasib (Form A) of the present invention as defined in any one of the above described aspects and their corresponding embodiments, a filler, a disintegrant and a lubricant.

In one embodiment, the filler is selected from the group consisting of dibasic calcium phosphate, kaolin, lactose monohydrate, dextrose, magnesium carbonate, sucrose, mannitol, glucose or other monosaccharides, dextrin or other polysaccharides, microcrystalline cellulose, powdered cellulose, cellulose derivatives, precipitated calcium carbonate, calcium sulfate, sorbitol, inositol and starch, and combinations thereof. Most preferably the filler is microcrystalline cellulose, lactose monohydrate or a combination thereof.

In one embodiment, the lubricant is selected from the group consisting of talc, magnesium stearate, calcium stearate, natrium stearate, glycerol monostearate, glyceryl tribehenate, sorbitan monostearate, sucrose monopalmitate, polyethylene glycol, boric acid, sodium oleate sodium benzoate, sodium acetate, sodium lauryl sulfate, magnesium lauryl sulfate, stearic acid, and combinations thereof. Most preferably the lubricant is magnesium stearate.

In yet another embodiment, the disintegrant is selected from the group consisting of croscarmellose sodium, crospovidone, sodium starch glycolate, starch (potato, corn), pregelatinized starch, microcrystalline cellulose, methyl cellulose, hydroxypropyl cellulose, sodium carboxy-methylcellulose and combinations thereof. Most preferably the disintegrant is croscarmellose sodium, microcrystalline cellulose or a combination thereof.

Preferably, the pharmaceutical composition of the present invention as defined in any one of the above described embodiments is an oral solid dosage form, more preferably a tablet or a capsule. In a particular preferred embodiment, the pharmaceutical composition of the present invention as described above is a capsule, preferably a hard gelatin capsule. Most preferably, the pharmaceutical composition of the present invention as described above is a tablet, preferably a film-coated tablet.

The pharmaceutical compositions of the present invention as defined in any one of the above described embodiments may be produced by standard manufacturing processes, which are well-known to the skilled person *e.g*. selected from the group consisting of micronization, blending, milling, granulation (wet or dry granulation), capsule filling, tabletting, film-coating and any combinations thereof.

In a particular aspect, the present invention relates to a process for the preparation of a pharmaceutical composition comprising the crystalline form of sotorasib (Form A) of the present invention, or the composition comprising the crystalline form of sotorasib (Form A) of the present invention as defined in any one of the above described embodiments, preferably a capsule such as a hard gelatin capsule, comprising the steps of:
(a) micronizing the crystalline form of sotorasib (Form A) of the present invention or the composition comprising the crystalline form of sotorasib (Form A) of the present invention;
(b) blending the micronized crystalline form of sotorasib (Form A) or the micronized composition comprising the crystalline form of sotorasib (Form A) of the present invention obtained in (a) with at least one filler and at least one lubricant;
(c) milling and/or sieving the blend obtained in (b);
(d) optionally, blending the milled and/or sieved mixture obtained in (c); and
(e) filling the mixture obtained in either (c) or (d) into capsules;

In another preferred aspect, the present invention relates to a process for the preparation of a pharmaceutical composition comprising the crystalline form of sotorasib (Form A) of the present invention, or the composition comprising the crystalline form of sotorasib (Form A) of the present invention as defined in any one of the above described embodiments, preferably a tablet such as a film-coated tablet, comprising the steps of:
(a) micronizing the crystalline form of sotorasib (Form A) of the present invention or the composition comprising the crystalline form of sotorasib (Form A) of the present invention;
(b) blending the micronized crystalline form of sotorasib (Form A) or the micronized composition comprising the crystalline form of sotorasib (Form A) of the present invention obtained in (a) with at least one filler and at least one lubricant;
(c) milling and/or sieving the blend obtained in (b);
(d) optionally, blending the milled and/or sieved mixture obtained in (c);
(e) pressing the mixture obtained in either (c) or (d) into tablets; and
(f) optionally film-coating the tablet cores;

In a further aspect, the present invention relates to the crystalline form of sotorasib (Form A) or the composition comprising the crystalline form of sotorasib (Form A), or the pharmaceutical composition comprising the crystalline form of sotorasib (Form A) or the composition comprising the crystalline form of sotorasib (Form A) as defined in any one of the above described aspects and their corresponding embodiments for use as a medicament.

In a further aspect, the present invention relates to the crystalline form of sotorasib (Form A), or the composition comprising the crystalline form of sotorasib (Form A), or the pharmaceutical composition comprising the crystalline form of sotorasib (Form A) or the composition comprising the crystalline form of sotorasib (Form A) as defined in any one of the above described aspects and their corresponding embodiments for use in the treatment of a cancer with a KRAS G12C mutation.

In another aspect, the present invention relates to a method of treating a cancer with a KRAS G12C mutation, the method comprising administering an effective amount of the crystalline form of sotorasib (Form A), or the composition comprising the crystalline form of sotorasib (Form A), or the pharmaceutical composition comprising the crystalline form of sotorasib (Form A) or the composition comprising the crystalline form of sotorasib (Form A) as defined in any one of the above described aspects and their corresponding embodiments to a patient in need of such a treatment.

In one embodiment, the cancer with a KRAS G12C mutation is selected from the group consisting of lung cancer, colorectal cancer and pancreatic cancer. In a preferred embodiment, the lung cancer is non-small cell lung cancer (NSCLC).

### EXAMPLES

The following non-limiting examples are illustrative for the disclosure and are not to be construed as to be in any way limiting for the scope of the invention.

### Example 1: Preparation of sotorasib Form A

Amorphous sotorasib (798 mg, *e.g.* prepared according to the procedure disclosed in Example 5 herein) was dissolved in isobutanol (5 mL) at room temperature. The resulting clear yellow solution was magnetically stirred at RT and turned into a colorless suspension after about 2 hours. After a total stirring time of 20 hours the solid was collected by filtration, washed with n-heptane and dried for 5 days at 40°C under vacuum (5 mbar) to obtain sotorasib Form A.
Yield: 620 mg

### Example 2: Preparation of sotorasib Form A

Amorphous sotorasib (763 mg, *e.g.* prepared according to the procedure disclosed in Example 5 herein) was suspended in MTBE (12 mL) at RT and stirred for 21 hours. The solid was collected by filtration and dried for 19 hours at 40°C under vacuum (5 mbar) to obtain sotorasib Form A.
Yield: 627 mg

### Example 3: Preparation of sotorasib Form A

Amorphous sotorasib (728 mg, *e.g.* prepared according to the procedure disclosed in Example 5 herein) was suspended in DIPE (14 mL) at RT and stirred for 72 hours. The solid was collected by filtration and dried for 41 hours at 40°C under vacuum (5 mbar) to obtain sotorasib Form A.
Yield: 609 mg

### Example 4: Preparation of sotorasib Form A

Sotorasib (9.40 g, e.g*.* prepared according to the procedure disclosed in Example 41 of WO 2018/217651 A1) was dissolved in methanol (317 g) at room temperature. Isobutanol (63 g) was added and the clear yellow solution was filtered. Methanol (5 mL) was added to the filtrate which was then transferred into a vacuum distillation equipment to remove methanol.

The distillation was performed by applying the conditions as specified in Table 1 below.

**Table 1: Distillation conditions**

| **time [min]** | **pressure [mbar]** | **T_{jacket} [°C]** | **Tₛᵤₘₚ [°C]** | **Tᵥₐₚₒᵤᵣ [°C]** |
|---|---|---|---|---|
| 0 | 120 | 23 | 19.0 | 22 |
| 2 | 120 | 55 | 19.0 | 22 |
| 4 | 120 | 55 | 20.9 | 22 |
| 7 | 120 | 55 | 21.3 | 22 |
| 22 | 120 | 55 | 21.5 | 22 |
| 47 | 120 | 55 | 21.6 | 22 |
| 62 | 120 | 55 | 21.8 | 22 |
| 77 | 120 | 55 | 22.1 | 22 |
| 102 | 120 | 55 | 22.3 | 22 |
| 129 | 120 | 55 | 29.5 | 22 |
| 144 | 120 | 55 | 40.1 | 22 |
| 151 | 120 | 55 | 43.8 | 22 |
| 159 | 120 | 55 | 45.9 | 22 |
| 160 | 120 | 20 | 45.0 | 22 |
| 204 | 120 | 20 | 26.0 | 22 |

The distillation was stopped by reducing the jacket temperature to 20°C when the sump temperature exceeded 45°C. Isobutanol (11.9 g) was added to the residual sump (35.6 g) to reach the desired crystallization concentration of about 20 w-% sotorasib. Subsequently, the solution was annealed by applying a jacket temperature of 20°C, seeded with sotorasib form A (2 mg, e.g. prepared according to the process disclosed in Example 1 herein) and stirred for 10 hours at 20°C. Then the temperature was linearly decreased to 0°C and further slurried at 0°C for 19 hours. The resulting precipitate was collected by filtration, washed twice with cold isobutanol (10 mL) and dried under vacuum for 90 min.
Yield: 7.73 g

### Example 5: Preparation of amorphous sotorasib

Sotorasib (3.075 g, e.g. prepared according to the procedure disclosed in Example 41 of WO 2018/217651 A1 ) was dissolved in methanol (200 mL). The clear solution was filtered and the solvent was removed by rotary evaporation at a bath temperature of 40 °C. The residue was further dried under vacuum (~ 5 mbar) at 40°C for 18 hours to yield amorphous sotorasib.
Yield: 2.751 g

### Example 6: Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

A representative diffractogram of sotorasib Form A of the present invention is displayed in Figure 1 hereinafter. The corresponding reflection list of crystalline form A of sotorasib of the present invention is provided in Table 2 below.

**Table 2: Reflection positions of crystalline Form A of sotorasib in the range of from 2 to 30° 2-Theta; a typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [° 2-Theta]** |
|---|---|---|---|
| 6.4 | 17.5 | 23.2 | 27.2 |
| 9.1 | 17.8 | 23.7 | 27.4 |
| 12.3 | 18.2 | 23.8 | 27.9 |
| 12.8 | 18.7 | 24.1 | 28.7 |
| 13.5 | 19.6 | 24.8 | 28.9 |
| 13.9 | 20.0 | 25.0 | 29.3 |
| 14.4 | 20.4 | 25.2 | 29.6 |
| 15.0 | 20.6 | 25.6 | 29.9 |
| 15.3 | 21.0 | 25.9 | |
| 16.0 | 22.7 | 26.4 | |
| 16.6 | 23.0 | 26.6 | |

### Example 7: Differential scanning calorimetry

DSC was performed on a Mettler Polymer DSC R instrument. The sample (3.31 mg Form A prepared according to Example 4 herein) was heated in a 40 microliter aluminium pan with a pierced aluminium lid from 25 to 350°C at a rate of 10° K/min. Nitrogen (purge rate 50 mL/min) was used as purge gas.

The DSC curve of sotorasib Form A prepared according to Example 4 herein is displayed in Figure 2 hereinafter and shows a first small endotherm with an onset temperature of about 159.2°C and a peak maximum at a temperature of about 172.3°C followed by a sharp endotherm with an onset temperature of about 285.3°C and a peak maximum at a temperature of about 287.8°C.

### Example 8: Thermogravimetric analysis

TGA was performed on a Mettler TGA/DSC 1 instrument. The sample (10.38 mg Form A prepared according to Example 4 herein) was heated in a 100 microliter aluminium pan closed with an aluminium lid from 25 to 350°C at a rate of 10 K/min. The lid was automatically pierced at the beginning of the measurement. Nitrogen (purge rate 50 mL/min) was used as purge gas.

The TGA curve of sotorasib Form A prepared according to Example 4 herein is displayed in Figure 3 hereinafter and shows a distinct step between about 120 and 240°C corresponding to a mass loss of about 1.8 w-% before the sample starts to decompose at higher temperature.

### Example 9: Gravimetric moisture sorption

Gravimetric moisture sorption was performed with an SPSx-1µ moisture sorption analyzer (ProUmid, Ulm). The measurement cycle was started at ambient relative humidity (RH) of 40%. RH was then decreased to 5% in 5% steps, followed by a further decrease to 3% and to 0%. Afterwards RH was increased from 0% to 90% in a sorption cycle and subsequently decreased to 0% in a desorption cycle each in 5% steps. Finally, RH was increased to ambient relative humidity of 40% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was (25 ± 0.1) °C.

The moisture sorption/desorption isotherms of sotorasib Form A of the present invention in the range from 0-90% RH are displayed in Figure 4. The mass difference in the sorption cycle between 0 and 90% RH is only about 0.4 w-% indicating the only slightly hygroscopic behaviour of this crystalline form. The lacking hysteresis between the sorption and the desorption isotherms indicates that no structural changes appear during the experiment. This assumption is strengthened by the fact that the sample still shows the same PXRD after the experiment.

### Example 10: Stress stability

Sotorasib Form A of the present invention was subjected to various temperature and humidity stress conditions for 30 weeks as outlined in Table 3 below. The samples were open stored in glass vials. The stressed samples were investigated by PXRD and as can be seen from the results summarized below no phase change occured at any stress condition.

**Table 3: Summary of the stress stability study**

| **25°C/60%RH** | **30°C/65%RH** | **40°C/75%RH** | **RT/97%RH** | **RT/0%RH** |
|---|---|---|---|---|
| Form A | Form A | Form A | Form A | Form A |

### Example 11: Powder blend comprising Sotorasib Form A

**Table 4: Composition of the final mixture**

| **Component** | **Target weight per tablet [mg]** | **Weight-%** | **Weight in batch size of 2.1 kg (3500 tablets) [kg]** |
|---|---|---|---|
| Sotorasib Form A | 120.0000 | 20.00 | 1.420 |
| Lactose Monohydrate | 120.0000 | 20.00 | 1.420 |
| Microcrystalline Cellulose (MMC) | 332.1450 | 55.40 | 1.163 |
| Croscarmellose sodium | 21.6650 | 3.61 | 0.076 |
| Magnesium stearate | 6.1900 | 1.03 | 0.022 |
| **Total** | **600.0000** | **100.00** | **2.100** |

The whole amount of microcrystalline cellulose was sieved through a 150 µm sieve. 50% of the sieved microcrystalline cellulose were mixed with the whole amount of lactose monohydrate and blended in a Turbulamixer ESD-TUMI-01 for 15 min at 34 rpm. The obtained mixture was sieved through a 150 µm sieve. After the sieving step, the remaining microcrystalline cellulose was added and the obtained mixture was blended in the Turbulamixer ESD-TUMI-01 for 15 min at 34 rpm. Then complete amounts of sotorasib Form A and croscarmellose sodium were mixed with the microcrystalline cellulose/lactose monohydrate mixture in three subsequent blending and sieving steps. First, approximately 25% of the microcrystalline cellulose/lactose monohydrate mixture were blended with Sotorasib Form A and crosscarmellose sodium in the Turbulamixer ESD-TUMI-01 for 5 min at 34 rpm. Next, the mixture was sieved through a 150 µm sieve before another 25% of the microcrystalline cellulose/lactose monohydrate mixture were added through a 150 µm sieve. The obtained mixture was blended in the Turbulamixer ESD-TUMI-01 for 5 min at 34 rpm and thereafter sieved through a 150 µm sieve. Then, the remaining amount (approximately 50%) of the microcrystalline cellulose/lactose monohydrate mixture was added to the mixture through a 150 µm sieve and the mixture was blended in Turbulamixer ESD-TUMI-01 for 15 min at 34 rpm.

Finally, magnesium stearate was added and the mixture was blended in the Turbulamixer ESD-TUMI-01 for 2 min at 34 rpm.

The thus obtained powder blend can either be pressed into tablet cores with 16mm x 6.5mm format by direct compression at 130 bar or filled into hard-gelain capsules of size 00.

### Comparative Example 1: Hygroscopicity

An analyses of the GMS data of sotorasib Form A of the present invention and anhydrous sotorasib Form I, Form II and Form III provided in WO 2020/236947 A1 reveals that Form A shows almost no interaction with water vapour and can be assigned as being slightly hygroscopic. According to the disclosure of WO 2020/236947 A1, page 46, paragraph [0334] Form I shows a mass increase of 0.5-1.0% in the range of 0-90% RH at 25°C, whereas Form II and Form III are hygroscopic materials both taking up well above 2 w-% of water, in the case of Form III even 7 w-% of water in the range of 0-90% RH at 25°C.

**Table 5: Summary of GMS data of the various sotorasib forms**

| **Selpercatinib** | **Mass increase from 0-90% RH at 25°C** |
|---|---|
| Form A of present invention (data from figure 4 of present invention) | + 0.4% |
| Form I of WO 2020/236947 A1 (data from paragraph [0334] on page 46) | +0.5 - 1.0% |
| Form II of WO 2020/236947 A1 (data from paragraph [0334] on page 46) | +2-2.5% |
| Form III of WO 2020/236947 A1 (data from paragraph [0334] on page 46) | +7.0% |

### Comparative Example 2: Dissolution rates of crystalline sotorasib Form I of WO 2020/236947 A1 and Form A of the present invention

Powder dissolution experiments were carried out at a temperature of 37°C in acetate buffer pH 4.5 and phosphate buffer pH 6.8 for the crystalline Form I of sotorasib disclosed in WO 2020/236947 A1 and the crystalline Form A of sotorasib of the present invention. Particle sizes of both forms were kept as similar as possible to allow for proper comparison. The respective concentrations were determined at a wavelength of 293 nm in case of the acetate buffer and at 354 nm in case of the phosphate buffer.

As can be seen from Figure 5 and Figure 6 hereinafter, sotorasib Form A of the present invention exhibits a significantly increased solubility in acetate buffer pH 4.5 and phosphate buffer pH 6.8 over the whole range from 15 minutes to 24 hours compared to the crystalline Form I of sotorasib disclosed in WO 2020/236947 A1. For example, the equilibrium solubility after 24 hours, is about 3-fold higher in acetate buffer and about 2-fold higher in phosphate buffer. PXRD confirmed that no phase changes orccured during the experiments.

## Claims

1. A crystalline form of sotorasib (Form A) according to the chemical structure as depicted in Formula (A) 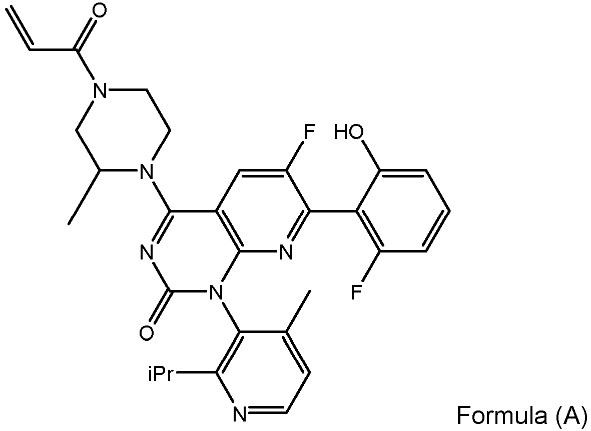 **characterized by** having a powder X-ray diffractogram comprising reflections at 2-Theta angles of (6.4 ± 0.2)°, (12.3 ± 0.2)°, (16.6 ± 0.2)° and (17.8 ± 0.2)°, when measured at a temperature in the range of from 20 to 30°C with Cu-Kalpha_{1,2} radiation having a wavelength of 0.15419 nm.

2. The crystalline form of claim 1 **characterized by** having a powder X-ray diffractogram comprising additional reflections at 2-Theta angles of (9.1 ± 0.2)° and/or (15.0 ± 0.2)°, when measured at a temperature in the range of from 20 to 30°C with Cu-Kalpha_{1,2} radiation having a wavelength of 0.15419 nm.

3. The crystalline form according to claim 1 or 2 **characterized by** having a powder X-ray diffractogram comprising no reflection at 2-Theta angles in the range of from 6.7° to 8.8°, when measured at a temperature in the range of from 20 to 30°C with Cu-Kalpha_{1,2} radiation having a wavelength of 0.15419 nm.

4. The crystalline form according to any one of the preceding claims **characterized by** having a powder X-ray diffractogram comprising no reflection at 2-Theta angles in the range of from 9.4° to 12.0°, when measured at a temperature in the range of from 20 to 30°C with Cu-Kalpha_{1,2} radiation having a wavelength of 0.15419 nm.

5. The crystalline form according to any one of the preceding claims **characterized by** showing a mass change of not more than 0.5 w-%, based on the weight of the crystalline form, when measured with gravimetric moisture sorption at a relative humidity in the range of from 0 to 90% and a temperature of (25.0 ± 1.0)°C.

6. The crystalline form according to any one of the preceding claims, wherein the form is the M atropisomer according to the chemical structure as depicted in Formula (A1)

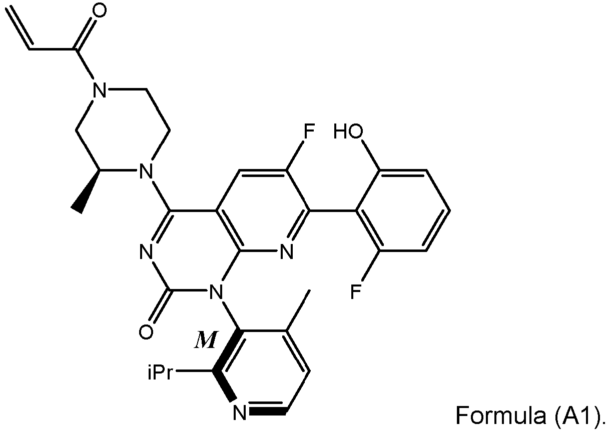

7. Use of the crystalline form as defined in any one of the preceding claims for the preparation of a pharmaceutical composition.

8. A pharmaceutical composition comprising a predetermined and/or effective amount of the crystalline form as defined in any one of claims 1 to 6 and at least one pharmaceutically acceptable excipient.

9. The pharmaceutical composition of claim 8, wherein the predetermined and/or effective amount is selected from the group consisting of 120 mg, 360 mg, 720 mg and 960 mg, calculated as anhydrous sotorasib.

10. The pharmaceutical composition according to claim 8 or 9, which is an oral solid dosage form, preferably wherein said oral solid dosage form is a capsule or a tablet.

11. The crystalline form as defined in any one of claims 1 to 6 or the pharmaceutical composition as defined in any one of claims 8 to 10 for use as a medicament.

12. The crystalline form as defined in any one of claims 1 to 6 or the pharmaceutical composition as defined in any one of claims 8 to 10 for use in the treatment of a cancer having a KRAS G12C mutation.

13. The crystalline form as defined in any one of claims 1 to 6 or the pharmaceutical composition as defined in any one of claims 8 to 10 for use according to claim 12, wherein the cancer having a KRAS G12C inhibition is selected from the group consisting of lung cancer, preferably the lung cancer is non-small cell lung cancer (NSCLC); colorectal cancer and pancreatic cancer.

14. A process for the preparation of the crystalline form of sotorasib as defined in any one of claims 1 to 6 comprising:
(a) dissolving or suspending amorphous sotorasib in a solvent comprising isobutanol, diisopropyl ether and/or methyl tert-butyl ether;
(b) crystallizing sotorasib Form A as defined in any one of claims 1 to 6 from the solution obtained in (a) at a temperature of 30°C or less;
(c) separating at least a part of the crystals obtained in (b) from the mother liquor;
(d) optionally washing the isolated crystals obtained in (c); and
(e) drying the crystals obtained in (c) or (d);

15. A process for the preparation of the crystalline form of sotoroasib as defined in any one of claims 1 to 6 comprising:
(a) dissolving sotorasib in a solvent comprising methanol;
(b) adding a solvent comprising isobutanol to the solution obtained in (a);
(c) removing the methanol from the solution obtained in (b);
(d) optionally seeding the mixture obtained in (c) with sotorasib Form A crystals as defined in any one of claims 1 to 6;
(e) crystallizing sotorasib Form A as defined in any one of claims 1 to 6 from the mixture obtained in (c) or (d) at a temperature of 30°C or less;
(f) separating at least a part of the crystals obtained in (e) from the mother liquor;
(g) optionally washing the isolated crystals obtained in (f); and
(h) drying the crystals obtained in (f) or (g);

## Patentansprüche

1. Kristalline Form von Sotorasib (Form A) gemäß der in Formel (A) dargestellten chemischen Struktur 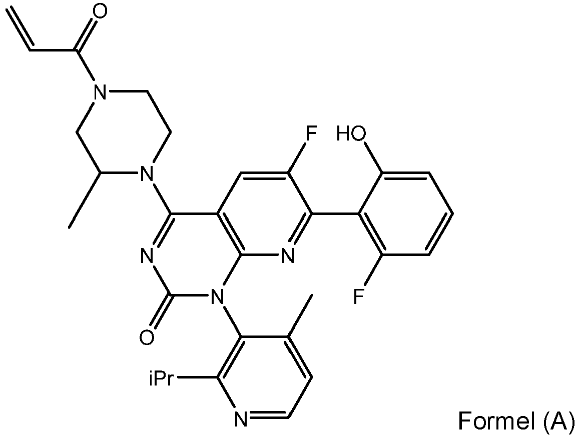 **gekennzeichnet durch** ein Pulver-Röntgendiffraktogramm, das Reflexionen bei 2-Theta-Winkeln von (6,4 ± 0,2)°, (12,3 ± 0,2)°, (16,6 ± 0,2)° und (17,8 ± 0,2)° umfasst, gemessen bei einer Temperatur im Bereich von 20 bis 30 °C mit Cu-Kalpha_{1,2}-Strahlung mit einer Wellenlänge von 0,15419 nm.

2. Kristalline Form von Anspruch 1, **gekennzeichnet durch** ein Pulver-Röntgendiffraktogramm, das zusätzliche Reflexionen bei 2-Theta-Winkeln von (9,1 ± 0,2)° und/oder (15,0 ± 0,2)° umfasst, gemessen bei einer Temperatur im Bereich von 20 bis 30 °C mit Cu-Kalpha_{1,2}-Strahlung mit einer Wellenlänge von 0,15419 nm.

3. Kristalline Form nach Anspruch 1 oder 2, **gekennzeichnet durch** ein Pulver-Röntgendiffraktogramm, das keine Reflexion bei 2-Theta-Winkeln im Bereich von 6,7° bis 8,8° umfasst, gemessen bei einer Temperatur im Bereich von 20 bis 30°C mit Cu-Kalpha_{1,2}-Strahlung mit einer Wellenlänge von 0,15419 nm.

4. Kristalline Form nach irgendeinem der vorhergehenden Ansprüche, **gekennzeichnet durch** ein Pulver-Röntgendiffraktogramm, das keine Reflexion bei 2-Theta-Winkeln im Bereich von 9,4° bis 12,0° umfasst, gemessen bei einer Temperatur im Bereich von 20 bis 30°C mit Cu-Kalpha_{1,2}-Strahlung mit einer Wellenlänge von 0,15419 nm.

5. Kristalline Form nach irgendeinem der vorhergehenden Ansprüche, **gekennzeichnet durch** Aufweisen einer Massenänderung von nicht mehr als 0,5 Gew.- %, bezogen auf das Gewicht der kristallinen Form, gemessen durch gravimetrische Feuchtigkeitssorption bei einer relativen Feuchtigkeit im Bereich von 0 bis 90 % und einer Temperatur von (25,0 ± 1,0) °C.

6. Kristalline Form nach irgendeinem der vorhergehenden Ansprüche, wobei die Form das M-Atropisomer gemäß der in Formel (A1) dargestellten chemischen Struktur ist

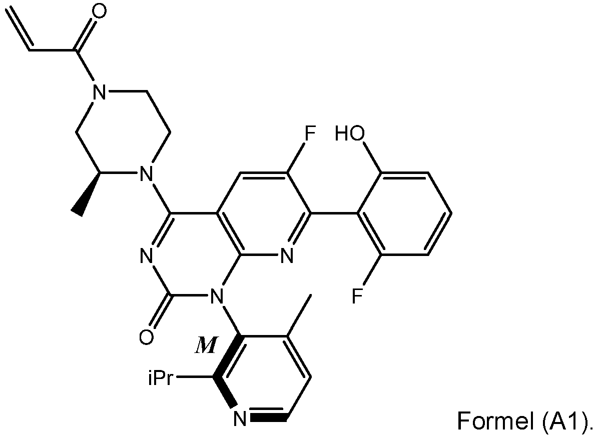

7. Verwendung der kristallinen Form, wie in irgendeinem der vorhergehenden Ansprüche definiert, zur Herstellung einer pharmazeutischen Zusammensetzung.

8. Pharmazeutische Zusammensetzung, umfassend eine vorbestimmte und/oder wirksame Menge der kristallinen Form, wie in irgendeinem der Ansprüche 1 bis 6 definiert, und mindestens einen pharmazeutisch akzeptablen Hilfsstoff.

9. Pharmazeutische Zusammensetzung nach Anspruch 8, wobei die vorbestimmte und/oder wirksame Menge aus der Gruppe ausgewählt ist, bestehend aus 120 mg, 360 mg, 720 mg und 960 mg, berechnet als wasserfreies Sotorasib.

10. Pharmazeutische Zusammensetzung nach Anspruch 8 oder 9, welche eine orale feste Dosierungsform ist, wobei die orale feste Dosierungsform bevorzugt eine Kapsel oder eine Tablette ist.

11. Kristalline Form, wie in irgendeinem der Ansprüche 1 bis 6 definiert, oder pharmazeutische Zusammensetzung, wie in irgendeinem der Ansprüche 8 bis 10 definiert, zur Verwendung als Medikament.

12. Kristalline Form, wie in irgendeinem der Ansprüche 1 bis 6 definiert, oder pharmazeutische Zusammensetzung, wie in irgendeinem der Ansprüche 8 bis 10 definiert, zur Verwendung bei der Behandlung eines Krebses mit einer KRAS-G12C-Mutation.

13. Kristalline Form, wie in irgendeinem der Ansprüche 1 bis 6 definiert, oder pharmazeutische Zusammensetzung, wie in irgendeinem der Ansprüche 8 bis 10 definiert, zur Verwendung nach Anspruch 12, wobei der Krebs mit einer KRAS-G12C-Inhibition ausgewählt ist aus der Gruppe bestehend aus Lungenkrebs, wobei der Lungenkrebs bevorzugt ein nicht-kleinzelliger Lungenkrebs (NSCLC) ist; kolorektalem Krebs und Bauchspeicheldrüsenkrebs.

14. Verfahren zur Herstellung der kristallinen Form von Sotorasib, wie in irgendeinem der Ansprüche 1 bis 6 definiert, umfassend:
(a) Auflösen oder Suspendieren von amorphem Sotorasib in einem Lösungsmittel, umfassend Isobutanol, Diisopropylether und/oder *tert-*Butylmethylether;
(b) Kristallisieren von Sotorasib Form A, wie in irgendeinem der Ansprüche 1 bis 6 definiert, aus der Lösung, die in (a) erhalten wurde, bei einer Temperatur von 30 °C oder weniger;
(c) Abtrennen mindestens eines Teils der Kristalle, die in (b) erhalten wurden, von der Mutterlauge;
(d) optional Waschen der isolierten Kristalle, die in (c) erhalten wurden; und
(e) Trocknen der Kristalle, die in (c) oder (d) erhalten wurden.

15. Verfahren zur Herstellung der kristallinen Form von Sotorasib, wie in irgendeinem der Ansprüche 1 bis 6 definiert, umfassend:
(a) Auflösen von Sotorasib in einem Lösungsmittel, umfassend Methanol;
(b) Zugeben eines Lösungsmittels, umfassend Isobutanol, zu der Lösung, die in (a) erhalten wurde;
(c) Entfernen des Methanols aus der Lösung, die in (b) erhalten wurde;
(d) optional Aussäen der in (c) erhaltenen Mischung mit Sotorasib-Form-A-Kristallen, wie in irgendeinem der Ansprüche 1 bis 6 definiert;
(e) Kristallisieren von Sotorasib Form A, wie in irgendeinem der Ansprüche 1 bis 6 definiert, aus der in (c) oder (d) erhaltenen Mischung bei einer Temperatur von 30 °C oder weniger;
(f) Abtrennen mindestens eines Teils der Kristalle, die in (e) erhalten wurden, von der Mutterlauge;
(g) optional Waschen der isolierten Kristalle, die in (f) erhalten wurden; und
(h) Trocknen der Kristalle, die in (f) oder (g) erhalten wurden.

## Revendications

1. Une forme cristalline de sotorasib (Forme A) selon la structure chimique telle que représentée dans la Formule (A) 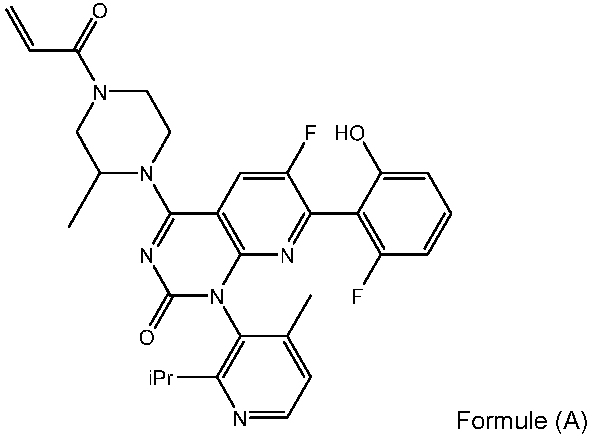 **caractérisée en ce qu'**elle possède un diffractogramme de rayons X sur poudre comprenant des réflexions à des angles 2-thêta de (6,4 ± 0,2)°, (12,3 ± 0,2)°, (16,6 ± 0,2)° et (17,8 ± 0,2)°, mesuré à une température comprise dans la gamme allant de 20 à 30°C avec un rayonnement Cu-Kalpha_{1,2} ayant une longueur d'onde de 0,15419 nm.

2. La forme cristalline de la revendication 1 **caractérisée en ce qu'**elle possède un diffractogramme de rayons X sur poudre comprenant des réflexions supplémentaires à des angles 2-thêta de (9,1 ± 0,2)° et/ou (15,0 ± 0,2)°, mesuré à une température comprise dans la gamme allant de 20 à 30°C avec un rayonnement Cu-Kalpha_{1,2} ayant une longueur d'onde de 0,15419 nm.

3. La forme cristalline selon la revendication 1 ou 2 **caractérisée en ce qu'**elle possède un diffractogramme de rayons X sur poudre ne comprenant aucune réflexion à des angles 2-thêta compris dans la gamme allant de 6,7° à 8,8°, mesuré à une température comprise dans la gamme allant de 20 à 30°C avec un rayonnement Cu-Kalpha_{1,2} ayant une longueur d'onde de 0,15419 nm.

4. La forme cristalline selon l'une quelconque des revendications précédentes **caractérisée en ce qu'**elle possède un diffractogramme de rayons X sur poudre ne comprenant aucune réflexion à des angles 2-thêta compris dans la gamme allant de 9,4° à 12,0°, mesuré à une température comprise dans la gamme allant de 20 à 30°C avec un rayonnement Cu-Kalpha_{1,2} ayant une longueur d'onde de 0,15419 nm.

5. La forme cristalline selon l'une quelconque des revendications précédentes **caractérisée en ce qu'**elle présente un changement de masse ne dépassant pas 0,5 % en poids, rapporté au poids de la forme cristalline, mesuré avec une sorption d'humidité gravimétrique à une humidité relative comprise dans la gamme allant de 0 à 90 % et à une température de (25,0 ± 1,0)°C.

6. La forme cristalline selon l'une quelconque des revendications précédentes, dans laquelle la forme est l'atropisomère M selon la structure chimique telle que représentée dans la Formule (A1)

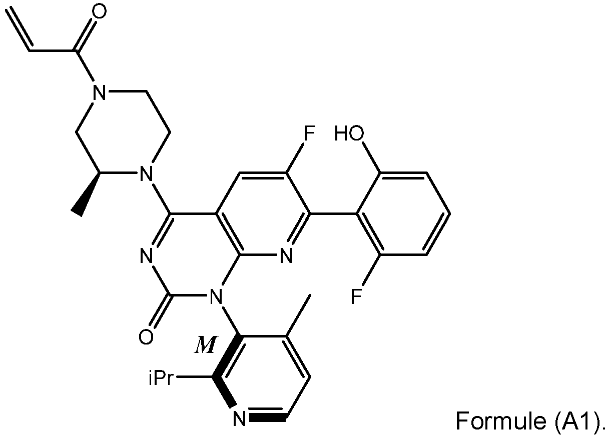

7. Utilisation de la forme cristalline telle que définie dans l'une quelconque des revendications précédentes pour la préparation d'une composition pharmaceutique.

8. Une composition pharmaceutique comprenant une quantité prédéterminée et/ou efficace de la forme cristalline telle que définie dans l'une quelconque des revendications 1 à 6 et au moins un excipient pharmaceutiquement acceptable.

9. La composition pharmaceutique de la revendication 8, dans laquelle la quantité prédéterminée et/ou efficace est sélectionnée dans le groupe constitué de 120 mg, 360 mg, 720 mg et 960 mg, calculé sous forme de sotorasib anhydre.

10. La composition pharmaceutique selon la revendication 8 ou 9, qui est une forme posologique solide orale, de préférence où ladite forme posologique solide orale est une capsule ou un comprimé.

11. La forme cristalline telle que définie dans l'une quelconque des revendications 1 à 6 ou la composition pharmaceutique telle que définie dans l'une quelconque des revendications 8 à 10 pour une utilisation en tant que médicament.

12. La forme cristalline telle que définie dans l'une quelconque des revendications 1 à 6 ou la composition pharmaceutique telle que définie dans l'une quelconque des revendications 8 à 10 pour une utilisation dans le traitement d'un cancer ayant une mutation de KRAS G12C.

13. La forme cristalline telle que définie dans l'une quelconque des revendications 1 à 6 ou la composition pharmaceutique telle que définie dans l'une quelconque des revendications 8 à 10 pour une utilisation selon la revendication 12, où le cancer ayant une inhibition de KRAS G12C est sélectionné dans le groupe constitué du cancer du poumon, de préférence le cancer du poumon est un cancer du poumon non à petites cellules (CPNPC) ; un cancer colorectal et un cancer du pancréas.

14. Un procédé pour la préparation de la forme cristalline de sotorasib telle que définie dans l'une quelconque des revendications 1 à 6 comprenant :
(a) dissoudre ou mettre en suspension du sotorasib amorphe dans un solvant comprenant de l'isobutanol, de l'éther diisopropylique et/ou de l'éther méthyl tert-butylique ;
(b) cristalliser la forme A de sotorasib telle que définie dans l'une quelconque des revendications 1 à 6 à partir de la solution obtenue en (a) à une température de 30°C ou moins ;
(c) séparer au moins une partie des cristaux obtenus en (b) de la liqueur mère ;
(d) facultativement laver les cristaux isolés obtenus en (c) ; et
(e) sécher les cristaux obtenus en (c) ou (d).

15. Un procédé pour la préparation de la forme cristalline de sotorasib telle que définie dans l'une quelconque des revendications 1 à 6 comprenant :
(a) dissoudre du sotorasib dans un solvant comprenant du méthanol ;
(b) ajouter un solvant comprenant de l'isobutanol à la solution obtenue en (a) ;
(c) éliminer le méthanol de la solution obtenue en (b) ;
(d) facultativement ensemencer le mélange obtenu en (c) avec des cristaux de sotorasib forme A tels que définis dans l'une quelconque des revendications 1 à 6 ;
(e) cristalliser la forme A de sotorasib telle que définie dans l'une quelconque des revendications 1 à 6 à partir du mélange obtenu en (c) ou (d) à une température de 30°C ou moins ;
(f) séparer au moins une partie des cristaux obtenus en (e) de la liqueur mère ;
(g) facultativement laver les cristaux isolés obtenus en (f) ; et
(h) sécher les cristaux obtenus en (f) ou (g) ;
